(19) Europäisches Patentamt
European Patent Office
Office européen des brevets

(11) **EP 4 029 026 B1**

(12) **EUROPEAN PATENT SPECIFICATION**

(45) Date of publication and mention
of the grant of the patent:
**23.07.2025 Bulletin 2025/30**

(21) Application number: **20761839.8**

(22) Date of filing: **26.08.2020**

(51) International Patent Classification (IPC):
**G16H 20/10** (2018.01)  **G16H 50/20** (2018.01)
**G09B 23/28** (2006.01)

(52) Cooperative Patent Classification (CPC):
**G09B 23/28; G16H 20/10; G16H 50/20**

(86) International application number:
**PCT/EP2020/073886**

(87) International publication number:
**WO 2021/047912 (18.03.2021 Gazette 2021/11)**

(54) **METHOD FOR TRAINING A MODEL USABLE TO COMPUTE AN INDEX OF NOCICEPTION**

VERFAHREN ZUM TRAINIEREN EINES ZUR BERECHNUNG EINES NOZIZEPTIONSINDEXES NÜTZLICHEN MODELLS

PROCÉDÉ DE FORMATION D'UN MODÈLE UTILISABLE POUR CALCULER UN INDICE DE NOCICEPTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.09.2019 EP 19382790**

(43) Date of publication of application:
**20.07.2022 Bulletin 2022/29**

(73) Proprietor: **QUANTIUM MEDICAL SLU**
**08302 Mataro (Catalunya) (ES)**

(72) Inventors:
• **GONZÁLEZ PIJUAN, Carmen**
**08302 Mataro (ES)**
• **MELIA, Umberto**
**08302 Mataro (ES)**
• **JENSEN, Erik**
**08302 Mataro (ES)**

(74) Representative: **Fresenius Kabi Deutschland GmbH**
**Patent Department - MedTech**
**Else-Kröner-Straße 1**
**61352 Bad Homburg (DE)**

(56) References cited:
**US-A1- 2018 206 784**

• **E. W. JENSEN ET AL: "Monitoring hypnotic effect and nociception with two EEG-derived indices, qCON and qNOX, during general anaesthesia : Monitoring anaesthesia with qCON and qNOX", ACTA ANAESTHESIOLOGICA SCANDINAVICA., vol. 58, no. 8, 4 July 2014 (2014-07-04), DK, pages 933 - 941, XP055500121, ISSN: 0001-5172, DOI: 10.1111/aas.12359**

**Description**

The invention relates to a method for training a model usable to compute an index of nociception related to a nociception effect during a general anesthesia procedure according to the preamble of claim 1, to a processing system, and to a monitoring device for computing an index of nociception related to a nociception effect during a general anesthesia procedure.

Within a method of this kind clinical data relating to a multiplicity of previous anesthesia procedures is obtained during a training phase separate from an actual use of the model during an anesthesia procedure. From the clinical data training data is derived. In addition, reference data is derived from the clinical data. Having obtained the training data and the reference data, the model is trained using the training data as input data to the model and the reference data as output data to the model, wherein during the training the model is adjusted according to the training data and the reference data.

Anesthesia is defined as a drug-induced state where the patient has lost consciousness, loss of sensation of pain or response to any other stimuli. To obtain these objectives, an anesthesiologist can use different classes of drugs, mostly hypnotics and analgesics, allowing patients to undergo surgery and other procedures without the distress and pain they would otherwise experience.

The state of analgesia for surgery is reached by the administration of analgesics. The demand of analgesics varies for each patient. Therefore there is a need for continuous, preferably non-invasive monitoring of the analgesia effect in the patient. Nociception and the perception of pain define the need for analgesia to obtain pain relief. Automatic responses such as tachycardia, hypertension, emotional sweating and lacrimation, although non-specific, are regarded as signs of nociception and consequently inadequate analgesia.

When administering a sufficient dose of hypnotics, the resulting loss of consciousness ensures that the patient does not perceive stimuli, but the neuro-vegetative and somatic responses are not necessarily abolished. When administering a sufficient dose of analgesics, nociception stimuli are blocked and neuro-vegetative and somatic responses are prevented. However, analgesics do not necessarily result in a loss of consciousness and amnesia.

Anesthesia generally can be regarded as a dynamic process where the effects of the anesthetic drugs are counteracted by the intensity of the different stimuli occurring during surgery. When an equilibrium resulting therefrom is broken, a patient could evolve to a different anesthetic depth, without the anesthesiologist being aware of it, resulting potentially in an intraoperative awareness of the patient. One of the objectives of modern anesthesia hence is to ensure adequate level of consciousness to prevent awareness without inadvertently overloading the patient with anesthetics, which might cause increased postoperative complications. There are several widely used clinical methods for assessing the level of consciousness during general anesthesia, including the Observer's Assessment of Alertness and Sedation scale (OAAS) and the Ramsey Sedation Scale. However, the disadvantages of using clinical scales in the operating room are that they cannot be used continuously and that they are cumbersome to perform. Furthermore, they require the patient's collaboration, which in some cases might be difficult. This has led to the investigation of an automated assessment of the level of consciousness.

Recently, some automatic devices have arrived on the market to provide an objective quantification of the level of consciousness of the patient. The most prevailing method is the analysis using an electroencephalogram (EEG) where a scalp EEG is recorded and subsequently processed by an algorithm which maps EEG signal into an index, typically in a range of 0 to 100. EEG-based methods are well-established for assessing brain activity by recording and analyzing the week bio-potential signals generated in the cortex of the brain with electrodes attached on the skin of the skull surface. It has been in wide use for decades in basic research of the neural systems of the brain, as well as in clinical diagnosis of various neurophysiological diseases and disorders.

A method for computing an index of nociception related to a nociception effect during a general anesthesia procedure making use of a model such as a quadratic model or a so-called ANFIS model (ANFIS stands for Adaptive Neuro Fuzzy Inference System) is for example described in an article by E. W. Jensen et al., "Monitoring hypnotic effect and nociception with two EEG-derived indices, qNOX and qCON, during general anesthesia", Acta Anaesthesiologica Scandinavica, 2014; 58:933- 941.

A method for computing an index of nociception is in addition disclosed in WO 2017/012622 A1.

The index of nociception, qNOX, is defined to indicate a probability of response to a noxious stimulus from low to high and may assume values in between 0 to 100. For computing the index of nociception a model is employed which, as input, uses EEG data and, as output, provides a value for the index of nociception, qNOX. The quality of the information provided by the index of nociception herein depends on the quality of the model, which accordingly during an initial training phase prior to the actual use of the model is trained by employing a large of amount of clinical data, for example relating to a multiplicity of anesthesia procedures carried out on multiple patients (for example hundreds of patients). From the clinical data, in particular training data relating to EEG data obtained during the several anesthesia procedures is used as input to the model, and reference data also derived from the clinical data is used as output, wherein the model during the training is adjusted such that the model reliably predicts values at least close to the reference data when using the training data is input.

The quality of the training hence depends on the quality of the reference data. There hence is a need to derive reference data which may be employed during a training of the model such that the model, at later use, is enabled to precisely predict an index of nociception which reflects the probability of response to noxious stimuli in a later anesthesia procedure.

It is an object of the instant invention to provide a method and a processing system for training a model usable to compute an index of nociception, the method and processing system enabling a suitable training of the model using training data and reference data.

It is another object of the instant invention to provide a monitor device for computing an index of nociception related to a nociception effect during a general anesthesia procedure making use of a model.

This object is achieved by a method comprising the features of claim 1.

Accordingly, the reference data is derived from the clinical data using an equation including a mathematical term whose value is nonlinearly variable as a function of a concentration value relating to a drug concentration in a patient during an anesthesia procedure.

In particular, the function of the concentration value is an exponential function.

The mathematical term is defined as *a·f1(CeRemi),* wherein a is a coefficient, and f1 defines said function, and *CeRemi* is said concentration value.

The concentration values may in particular relate to an effect-site concentration of remifentanil used as an analgesic during an anesthesia procedure.

The clinical data is obtained from a multiplicity of previous anesthesia procedures. Such previous anesthesia procedures may for example have been carried out using a target-controlled infusion (TCI) as described for example in WO 2014/173558 A1. During such anesthesia procedures a vast amount of data relating to the anesthesia procedures is recorded, such as EEG data, data relating to events (such as stimulation events during surgery and the like), and concentration values in different compartments of the patient (as computed for example within a target-controlled infusion), in particular also an effect-site concentration for example in the patient's brain is obtained during the anesthesia. This clinical data, which has been obtained previously and is available for training the model, is used to derive the training data and the reference data.

Herein, the reference data shall indicate an actual index of nociception which shall (at closely) be output by the model when feeding the model with corresponding training data. The training data herein relates to in particular EEG data as it later on, during an actual anesthesia procedure, shall be fed to the model. The reference data is derived from additional data of the clinical data correlated to the EEG data.

For deriving the reference data, it herein has been found that a concentration value relating to a drug concentration in a patient, for example an analgesic concentration, in particular the effect-site concentration of the analgesic in the patient's brain, should be taken into account in a nonlinear fashion by using a nonlinear function for computing the reference data. The function particular may be an exponential function, causing low values of the concentration value to substantially increase the value of the reference, wherein the function nonlinearly decays with increasing concentration values such that a large concentration value causes a near-zero contribution to the reference.

By employing a nonlinear function for taking the concentration value into account for deriving the reference data, a more reliable reference may be obtained for training the model.

In particular, for computing the reference data the following equation is employed:

$$RD = a·f1(CeRemi) + b·qCON + f3(Resp)$$

wherein RD is the reference data, a, *b* are coefficients, qCON is an index of consciousness, f3 is a function and *Resp* is a patient response parameter. qCON is computed also from the clinical data using another model, for example as described in WO 2017/012622 A1. *Resp* is a parameter reflecting the intensity of movement as a consequence of a stimulation.

*f3(Resp)* may for example computed using the following equation:

$$f3(Resp) = \begin{cases} 15 * Resp \text{ if } Resp > 0 \text{ and } t = 60 \text{ } s \text{ before and after annotation} \\ -40 \text{ if } Resp = 0 \text{ and } t = 60 \text{ } s \text{ before and after annotation} \\ 0 \text{ } Otherwise \text{ (unknown or no annotation)} \end{cases}$$

where

$$Resp = \begin{cases} 5 \text{ if Total Movement } - \text{ patient awake, talking.} \\ 4 \text{ if Movement just after a stimulus} \\ 3 \text{ if Intense movement whole body movements, open eyes, pain,} \\ \text{yawning, coughing} \\ 2 \text{ if Medium movement } - \text{ tremor, hiccup, hand movement} \\ 1 \text{ if Low movement } - \text{ eye movement} \\ 0 \text{ No movement after a stimulus} \end{cases}$$

For deriving the reference data, the coefficients a, *b* may be iteratively set by experimentation. For example, the coefficients may be initially set to a particular set of values, wherein the model is trained using the reference data obtained in this way. The coefficients may then be adjusted to improve the training of the model. This may be iterated multiple times.

The clinical data relates to multiple previous anesthesia procedures. Within the anesthesia procedures, herein, the concentration value generally is time-variable, reflecting the anesthesia process by infusing a time-variable dose of a suitable anesthetic, such as remifentanil or propofol. Herein, for each of the anesthesia procedures for which clinical data is obtained a time-variable reference curve may be computed, such that the model may be trained by applying the reference curves and corresponding EEG data relating to the anesthesia procedures as stored in the clinical data.

Each of the reference curves herein may be scaled to fall into a range between 0 and 100 and/or smoothed by applying a moving averaging technique, in particular an exponential moving average and (for example with a 0.9 factor for previous values and a 0.1 factor for new values).

The model used to compute the index of nociception may in particular be a fuzzy logic model (in particular a so-called ANFIS model) or a quadratic model, as for example described in WO 2017/012622 A1. The model in particular may be defined by a system of equations using a multiplicity of coefficients for computing said index of nociception from input data derived from an encephalography signal (EEG), wherein during the training the training data is used as input data and the coefficients are adjusted to define the model. Once the training is completed the model is frozen and may be used, by a monitor device, during an actual anesthesia procedure to compute the index of nociception during the anesthesia procedure in real time to provide an information with respect to nociception during general anesthesia.

A processing system configured to execute a software code implementing the method as described above may be, for example, separate from a monitor device. Such processing system may for example be a general computer system located at the site of a developer of a monitor device, wherein upon completion of the model the model may be installed on the monitor device and may be used, during an anesthesia procedure, on-site at a hospital to monitor the anesthesia procedure.

A monitor device for computing an index of nociception related to a nociception effect during a general anesthesia procedure comprises a processor device configured to compute said index of nociception during an actual anesthesia procedure using a model and input data derived from an encephalography (EEG) signal obtained during the general anesthesia procedure, wherein a value for the index of nociception is obtained as output from the model. The processor device herein is configured to modify the value for the index of nociception as obtained from the model using additional information derived from the encephalography (EEG) signal to obtain a corrected value for the index of nociception.

The model provides a measure for the index of nociception. Herein, to further improve the reliability of the index of nociception and its ability to predict a probability for a patient's response to a stimulus during surgery, after receiving the value for the index of nociception from the model the value is corrected by taking additional information also derived from the EEG signal into account.

The processor device may in particular be constituted to compute the value for the index of nociception in real-time during the actual anesthesia procedure. The monitor device may for example comprise a display on which the (corrected) value for the index of nociception is displayed in real-time during the anesthesia procedure to indicate an actual probability for a patient's responsiveness to stimuli.

The processor device is configured to modify the value for the index of nociception obtained from the model by using information related to at least one of an electrooculogram derived from the encephalography signal, a burst suppression ratio derived from the encephalography signal, and a near burst suppression index derived from the encephalography signal.

An electrooculogram may for example be calculated from an EEG signal as the amount (count) of peaks that are above a certain amplitude threshold in a window of the EEG signal. The amount of the EOG signal derived from the EEG signal is related to patient wakefulness, such that a high value for the EOG may be used to correct the index of nociception to a higher value.

The burst suppression ratio relates to deep anesthesia and reflects a ratio of blank periods in the EEG signal to periods of fast activity in the EEG signal. The burst suppression ratio may be used to correct the value for the index of nociception using the following equations:

$$qNOX\mathrm{cor}=\max(0,1-BSR/30)*qNOX+\min(1,BSR/30)*(41-0.41\ BSR)$$

where *qNOXcor* is the corrected value for the index of nociception, max() and min() correspond to the maximum and minimum operators, respectively, and BSR is the burst suppression ratio. As it can be observed, for BSR values of 30 or more, the value of the qNOX index only depends on the BSR parameter. The BSR correction, in one embodiment, is limited only to decrease the qNOX value. An increase of the value of the qNOX due to burst suppression generally is not desired.

The near burst suppression index (NBS) indicates whether the EEG exhibits a pattern similar to burst suppression, but in which the signal amplitude between bursts is not low enough to be considered as suppressed EEG. This pattern often appears slightly before burst suppression is detected. The near burst suppression index may for example be computed as the standard deviation of the energy in the frequency band 11-22 Hz over 10s intervals. The output of the qNOX after the BSR correction may, in one embodiment, be modified depending on the near burst suppression parameter.

In one embodiment, an exponential correction is used to decrease the qNOX value during near burst suppression periods. The NBS correction is limited only to decrease the qNOX value. The correction is applied for values of NBS parameter higher than 0.8 and with an EOG count less than 60.

The value for the index of nociception may be corrected according to the near burst suppression index for example making use of the following formula:

$$qNOX=\exp(a+b*qNOX-cNBS)$$

where *cNBS* is the near burst suppression index, and a, *b* are coefficients which are defined experimentally.

In one embodiment, in addition to a modification of the index of nociception (as output by the model) by taking information derived from the EEG signal into account, the value for the index of nociception may further be modified by applying at least one of a scaling operation and a smoothing operation. Within the scaling, the value for the index of nociception is scaled such that it falls for example into a range between 25 and 100. The scaling may for example be carried out by using the following formula system:

$$qNOX=1.3751*qNOX-28.7514\ for\ 80\leq qNOX<94$$
$$qNOX=0.4*qNOX+21\ for\ 15\leq qNOX<35$$
$$qNOX=0.1333*qNOX+25\ for\ qNOX<15$$

The model as used for computing the index of nociception may in particular be a model as defined, in an initial training phase, making use of the method as described above for training the model.

The idea underlying the invention shall subsequently be described in more detail by referring to the embodiments shown in the figures. Herein:

Fig. 1        shows a schematic view of a setup in an anesthesia procedure;
Fig. 2        shows a functional diagram of the setup of Fig. 1;
Fig. 3        shows a functional diagram of a model for modelling the distribution of a drug dosage in a patient's body;
Fig. 4        shows a schematic drawing of a model for computing an index of consciousness (qCON);
Fig. 5        shows a schematic drawing of a model for computing an index of nociception (qNOX);
Fig. 6        shows a schematic drawing for a correction of a value for the index of nociception as output by the model;
Fig. 7        shows a schematic drawing during training of the model for computing the index of nociception;
Fig. 8        shows a graph for a function for taking a concentration value into account for deriving reference data from clinical data;
Fig. 9        shows a schematic drawing of a monitor device employing models for computing an index of consciousness and an index of nociception;
Fig. 10        shows a qualitative graph showing an index of nociception and a concentration during an anesthesia procedure; and
Fig. 11A, 11B        show a mathematical formulation of an ANFIS non-linear model.

Subsequently, a method and processing system for defining a model usable to compute an index of nociception related to a nociception effect during a general anesthesia procedure as well as a monitor device for computing an index of nociception during an actual anesthesia procedure shall be described in certain embodiments. The embodiments described herein shall not be construed as limiting for the scope of the invention.

Like reference numerals are used throughout the figures as appropriate.

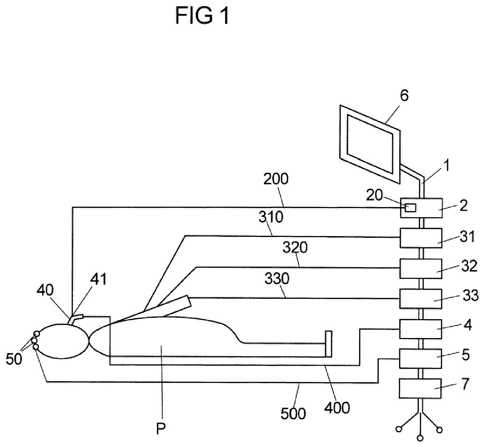

A model to compute an index of nociception may generally be used during a general anesthesia procedure, for example an anesthesia procedure making use of a target-controlled infusion (TCI), which shall subsequently be described according to Figs. 1 to 3. Making use of a model the index of nociception may however be computed generally in any general anesthesia procedure from EEG input data.

Fig. 1 shows a schematic drawing of a setup as it generally is used for example in an anesthesia procedure for administering an anaesthetic drug such as propofol and/or remifentanil to a patient P. In this setup multiple devices are arranged on a rack 1 and are connected via different lines to the patient P.

In particular, infusion devices 31, 32, 33 such as infusion pumps, in particular syringe pumps or volumetric pumps, are connected to the patient P and serve to intravenously inject, via lines 310, 320, 330, different drugs such as propofol, remifentanil and/or a muscle relaxant drug to the patient P in order to achieve a desired anaesthetic effect. The lines 310, 320, 330 are for example connected to a single port providing access to the venous system of the patient P such that via the lines 310, 320, 330 the respective drugs can be injected into the patient's venous system.

The rack 1 furthermore holds a ventilation device 4 for providing an artificial respiration to the patient P while the patient P is under anesthesia. The ventilation device 4 is connected via a line 400 to a mouth piece 40 such that it is in connection with the respiratory system of the patient P.

The rack 1 also holds an EEG monitor 5 which is connected via a line or a bundle of lines 500 to electrodes 50 attached to the patient's head for monitoring the patient's brain activity during an anesthesia procedure.

In addition, a control device 2 is held by the rack 1 which comprises a measurement device 20 connected to a junction 41 of the mouth piece 40 via a line 200. The control device 2 serves to control the infusion operation of one or multiple of the infusion devices 31, 32, 33 during the anesthesia procedure such that infusion devices 31, 32, 33 inject anaesthetic drugs to the patient P in a controlled fashion to obtain a desired anaesthetic effect. This shall be explained in more detail below.

The measurement device 20 serves to measure the concentration of one or multiple anaesthetic drugs in the breath of the patient P. The measurement device 20 may for example measure the propofol concentration in the patient's P exhaled breath. The measurement device 20, for this, may for example continuously measure over a predefined number of breathing cycles (inhalation and exhalation), for example six breathing cycles, in order to then suitably average the measured concentration in the patient's P breath over the breathing cycles. Alternatively, the measurement device 20 may also measure the concentration of for example propofol only during exhalation phases, wherein a suitable triggering mechanism for triggering the measurement may be used, or it continuously measures the propofol concentration.

The control device 2 may be adapted to provide information about a measured drug concentration in the patient's P breath or in other compartments of the patient P, or information about the drug effect in patient's brain compartment. Such information can be output via a monitor 6 attached to the rack 1 such that personnel, such as an anaesthesiologist, may monitor a drug concentration and the related effect achieved in the patient P during an anesthesia procedure.

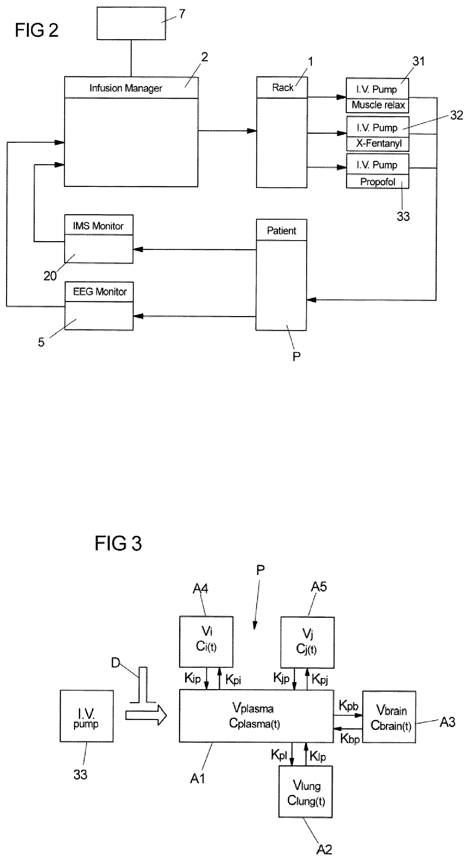

Fig. 2 shows a functional diagram of a control loop for controlling the infusion operation of the infusion devices 31, 32, 33 during an anesthesia procedure. The control loop herein may in principle be set up as a closed loop in which the operation of the infusion devices 31, 32, 33 is automatically controlled without user interaction. Beneficially, however, the system is set up as an open-loop system in which at certain points of time, in particular prior to administering a drug dosage to a patient, a user interaction is required in order to manually confirm the operation.

The control device 2, also denoted as "infusion manager", is connected to the rack 1 which serves as a communication link to the infusion devices 31, 32, 33 also attached to the rack 1. The control device 2 outputs control signals to control the operation of the infusion devices 31, 32, 33, which according to the received control signals inject defined dosages of drugs to the patient P.

By means of the EEG monitor 5 an EEG reading of the patient P is taken, and by means of the measurement device 20 the concentration of one or multiple drugs in the patient's P breath is measured. The measured data obtained by the EEG monitor 5 and the measurement device 20 are fed back to the control device 2, which correspondingly adjusts its control operation and outputs modified control signals to the infusion devices 31, 32, 33 to achieve a desired anaesthetic effect.

The measurement device 20 may for example be constituted by a so called IMS monitor for measuring a drug concentration in the patient's P breath by means of the so called Ion Mobility Spectrometry. Other sensor technologies may also be used.

The control device 2 uses, to control the infusion operation of one or multiple infusion devices 31, 32, 33, a pharmacokinetic-pharmacodynamic (PK/PD) model, which is a pharmacological model for modelling processes acting on a drug in the patient's P body. Such processes include the resorption, the distribution, the biochemical metabolism and the excretion of the drug in the patient's P body (denoted as pharmacokinetics) as well as the effects of a drug in an organism (denoted as pharmacodynamics). Preferably, a physiological PK/PD model with N compartments is used for which the transfer rate coefficients have been experimentally measured beforehand (for example in a proband study) and are hence known. To simplify the PK/PD model not more than 4-5 compartments preferably are used.

A schematic functional drawing of the setup of such a PK/PD model p is shown in Fig. 3. The PK/PD model p logically divides the patient P into different compartments A1-A5, for example a plasma compartment A1 corresponding to the patient's P blood stream, a lung compartment A2 corresponding to the patient's P lung, a brain compartment A3 corresponding to the patient's P brain and other compartments A4, A5 corresponding, for example, to muscular tissue or fat and connective tissue. The PK/PD model p takes into account the volume $V_{Lung}$, $V_{plasma}$, $V_{brain}$, $V_i$, $V_j$ of the different compartments A1-A5 as well as transfer rate constants $K_{PL}$, $K_{LP}$, $K_{BP}$, $K_{PB}$, $K_{IP}$; $K_{PI}$, $K_{JP}$, $K_{PJ}$ indicating the transfer rates between the plasma compartment A1 and the other compartments A2-A5, assuming that a drug dosage D by means on an infusion device 33 is injected into the plasma compartment A1 and the plasma compartment A1 links the other compartments A2-A5 such that an exchange between the other compartments A2-A5 always takes place via the plasma compartment A1. The PK/PD model p serves to predict the concentration $C_{lung}$, $C_{plasma}$, $C_{brain}$, $C_i$, $C_j$ of the injected drug in the different compartments A1-A5 as a function of time.

During a general anesthesia procedure, carried out for example by using a control device 2 and a control in the sense of a target-controlled infusion (TCI) as described above, it generally is desired to be able to provide for an accurate assessment of an anesthetic state of a patient. For this, from information obtained during a general anesthesia procedure, in particular EEG signals obtained from the EEG monitor 5, indices shall be computed reflecting a level of consciousness and a level of nociception of a patient during the anesthesia procedure.

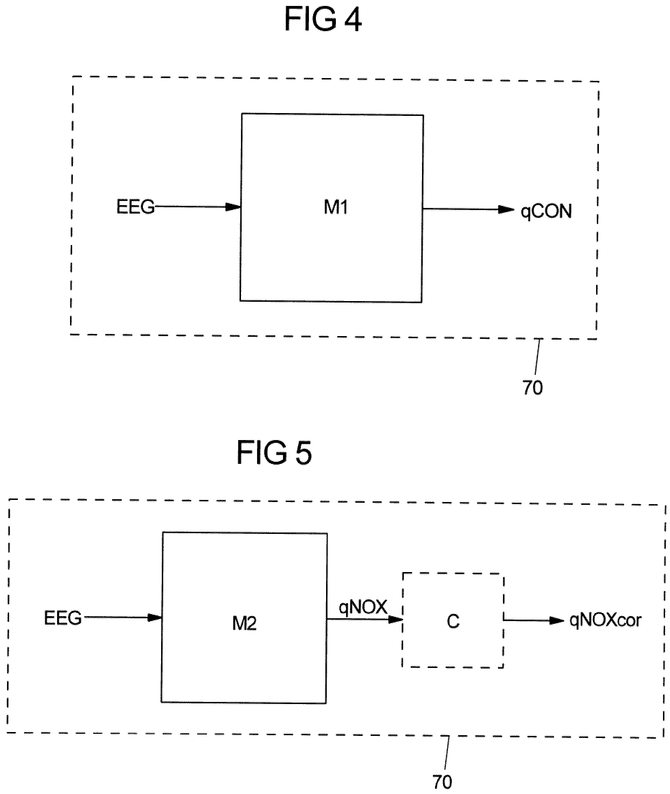

This is schematically illustrated in Figs. 4 and 5. Namely, based on an EEG signal obtained during a general anesthesia procedure a first model M1 is used to compute an index of consciousness qCON (Fig. 4), and a second model M2 is used to compute an index of nociception qNOX (Fig. 5), as this is described for example in WO 2017/012622 A1. During a general anesthesia procedure, hence, the models M1, M2 are used to compute from an input EEG signal an index of consciousness qCON reflecting a level of consciousness, and an index of nociception qNOX reflecting a probability of response to noxious stimuli. Both indices generally have values ranging from 0 to 100 (where higher values indicate an increased consciousness and nociception, respectively).

For computing the index of nociception qNOX, EEG data is fed to the model M2, and a value for the index of nociception qNOX is obtained as output from the model M2. Herein, in addition it is proposed to employ a correction function C for correcting the value for the index of nociception qNOX to obtain a corrected value qNOXcor for the index of nociception.

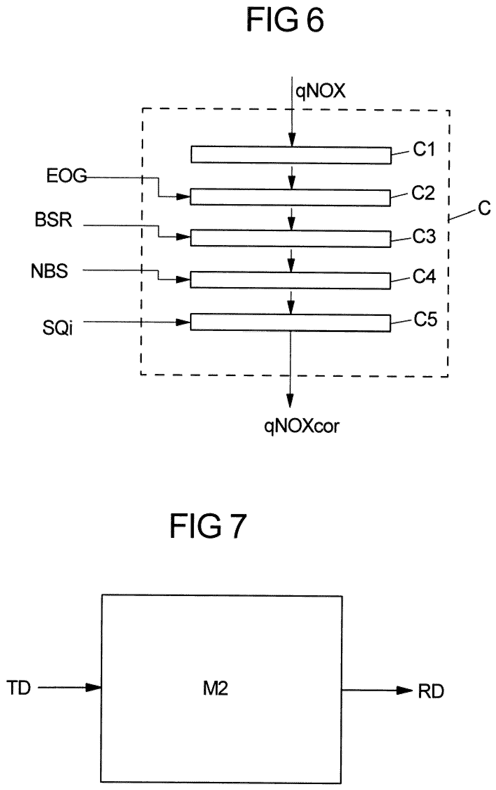

Referring now to Fig. 6, for correcting the index of nociception different correction functions C1-C5 may be employed.

Namely, in a first correction function C1 a scaling may be employed to scale the value for the index of nociception to values between 25 and 99, values below 25 hence not being allowed. For the scaling, for example the following equations may be used:

$$qNOX = 1.3751 * qNOX - 28.7514 \; for \; 80 \leq qNOX < 94$$
$$qNOX = 0.4 * qNOX + 21 \; for \; 15 \leq qNOX < 35$$
$$qNOX = 0.1333 * qNOX + 25 \; for \; qNOX < 15$$

In a second correction function C2 a correction according to a so-called electrooculogram EOG may be carried out, the electrooculogram EOG being computed from the EEG signal as the count of peaks above a certain threshold within a window of the EEG signal. A large count of EOG may indicate an increased wakefulness, which may be used to correct the index of nociception.

In a third correction function C3 a burst suppression ratio, indicating a state of deep anesthesia, may be taken into account to correct the value for the index of nociception in particular to lower values. Within the correction function C3 in particular the following equation may be employed:

$$qNOXcor = max(0, 1 - BSR/30) * qNOX + min(1, BSR/30) * (41 - 0.41 \; BSR)$$

where $qNOXcor$ is the corrected value for the index of nociception, max() and min() correspond to the maximum and minimum operators, respectively, and BSR is the burst suppression ratio. As it can be observed, for BSR values of 30 or more, the value of the qNOX index only depends on the BSR parameter. The BSR correction, in one embodiment, is limited only to decrease the qNOX value. An increase of the value of the qNOX due to burst suppression generally is not desired.

In a fourth correction function C4 a near burst suppression index NBS may be used to correct the value for the index of nociception. The near burst suppression index indicates whether the EEG exhibits a pattern similar to burst suppression, but in which the signal amplitude between bursts is not low enough to be considered as suppressed EEG. This pattern often appears slightly before burst suppression is detected. The near burst suppression index may for example be computed as the standard deviation of the energy in the frequency band 11-22 Hz over 10s intervals. The output of the qNOX after the BSR correction may, in one embodiment, be modified depending on the near burst suppression parameter.

For example an exponential correction is used to decrease the qNOX value during near burst suppression periods making use of the following formula:

$$qNOX = \exp(a + b * qNOX - cNBS)$$

where *cNBS* is the actual value for the near burst suppression index, and a, *b* are coefficients which are defined experimentally.

In a fifth correction function C5 a smoothing may be employed, for example according to a signal quality index SQI. The smoothing may for example be carried out by a running average or the like.

The model M2 to compute the index of nociception may for example be a quadratic model or a fuzzy logic model, in particular an ANFIS model as shall be subsequently be described in more detail according to different examples. Generally, the model M2 may be represented by a system of equations comprising a multiplicity of coefficients, which are suitably defined in an initial training phase by training the model M2 such that the model M2 reliably provides an output for the index of nociception when feed with input data derived from the EEG signal, as illustrated in Fig. 5.

The training, as shown in Fig. 7, herein generally takes place by using training data TD as input and reference data RD as output, wherein the model M2 is fitted to the training data TD and the reference data RD in an adjustment process using a large amount of clinical data for deriving the training data TD and the reference data RD.

Generally, clinical data is obtained during anesthesia procedures, for example by carrying out general anesthesia procedures using a target-controlled infusion (TCI) as described above according to Figs. 1 to 3. During such anesthesia procedures data is recorded relating for example to the EEG signal, to concentration values in the different compartments as obtained from the PK/PD model, to events occurring during an operation, such as surgery events (for example the time of an incision or the like representing a stimulus), to the patient's state such as signs of wakefulness and the like, and to all other data available during an anesthesia procedure. Hence, within the clinical data an EEG signal is linked to the actual drug concentration in the different compartments of the patient (in particular also at the effect site, generally the brain, of the patient), and the patient's state and response to stimuli occurring during surgery.

Hence, from the clinical data a reference can be derived which is the target output which should be yielded by the model M2 as value for the index of nociception.

It herein is proposed to use the following equation for deriving the reference data for the index of nociception for training the model:

$$RD = a \cdot f1(CeRemi) + b \cdot qCON + f3(Resp)$$

wherein a, *b* are coefficients, *f1* is an exponential function, *CeRemi* is the effect-site concentration of the infused anesthetic drug (e.g., remifentanil), *qCON* is an index of consciousness, *f3* is a function and *Resp* is a patient response parameter.

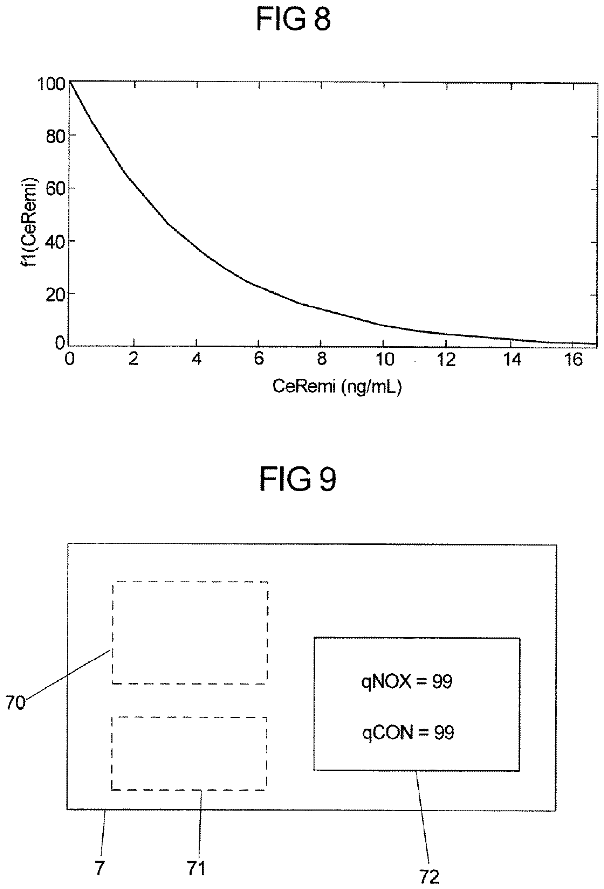

The exponential function *f1* is illustrated in Fig. 8.

qCON is computed also from the clinical data using the model M1, for example as described in WO 2017/012622 A1.

*Resp* is a parameter reflecting the intensity of movement as a consequence of a stimulation.

*f3(Resp)* may for example computed using the following equation:

$$f3(Resp) = \begin{cases} 15 * Resp \ if \ Resp > 0 \ and \ t = 60 \ s \ before \ and \ after \ annotation \\ -40 \ if \ Resp = 0 \ and \ t = 60 \ s \ before \ and \ after \ annotation \\ 0 \ Otherwise \ (unknown \ or \ no \ annotation) \end{cases}$$

where

$$Resp = \begin{cases} 5 \ if \ Total \ Movement - patient \ awake, talking. \\ 4 \ if \ Movement \ just \ after \ a \ stimulus \\ 3 \ if \ Intense \ movement \ whole \ body \ movements, open \ eyes, pain, \\ yawning, coughing \\ 2 \ if \ Medium \ movement - tremor, hiccup, hand \ movement \\ 1 \ if \ Low \ movement - eye \ movement \\ 0 \ No \ movement \ after \ a \ stimulus \end{cases}$$

As another option, the reference data RD may be defined using the following equation:

$$Ref = ARef + RqCON$$

Herein, ARef indicates the difference between the maximum drug dosage and the actual drug dosage at each time interval. RqCON is derived from the qCON index, wherein for values between 0 and 20 RqCON is set to 0, for values between 20 and 40 RqCON is set to 1, for values between 40 and 60 RqCON is set to 2, for values between 60 and 80 RqCON is set to 3, and for values between 80 and 100 RqCON is set to 4.

In addition, herein, an event of noxious stimulation may be taking into account, in that in case a noxious stimulation occurs and results in a patient movement the reference is set to 10, otherwise it remains at the value provided by the above equation.

To obtain the reference, the values obtained in this way are scaled to a range between 0 and 100 by miultiplying the obtained value by a factor of 10.

Using the reference data RD as derived in this way, the model M2 is trained. The reference data RD herein generally is provided as reference curves which are time variable, each reference curve relating to a particular anesthesia procedure and being correlated with EEG data obtained during that anesthesia procedure. Hence the training data is derived from the corresponding EEG data, and the model is trained such that the model M2 (at least closely) predicts the reference data RD when fed with the training data TD. This is applied to a vast amount of clinical data, relating to for example hundreds of patients, such that the model M2 is fitted to be able to reliably predict a value for the index of nociception when fed with input data derived from an EEG signal.

Once the training is completed, the model M2 is frozen and is installed on a monitor device 7 for use in an actual anesthesia procedure, as this is shown in the setup of Figs. 1 and 2. The monitor device 7 is functionally linked to the EEG monitor 5 and hence, during an actual anesthesia procedure, may receive EEG data as input, such that the index of nociception qNOX may be computed in real-time during the anesthesia procedure.

As illustrated in Fig. 9, the monitor device 7 comprises a processor device 17 and a memory device 71. The model M1 for computing the index of consciousness qCON and the model M2 for computing the index of nociception qNOX are the stored on the memory device 71 and are used during an anesthesia procedure to in real-time compute corresponding indices. The monitor device 7 for example comprises a display device 72, such that the corresponding indices may in real-time be displayed to a user.

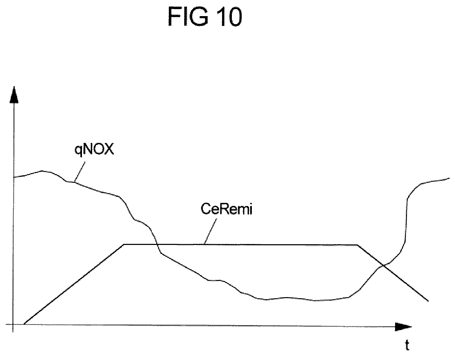

Fig. 10 illustrates a curve for the index of nociception qNOX over time and a corresponding curve for the concentration of drug at the effect site, for example the concentration of remifentanil in the patient's brain as computed for example by employing a PK/PD model during a TCI anesthesia procedure. When deriving reference data from clinical data, the reference data may have the shape of the qNOX curve as illustrated in Fig. 10.

The training of the non-linear model is beneficially carried out with a large amount of clinical data obtained during multiple previous anesthesia procedures, in particular TCI anesthesia procedures. The training, which is done in an initial pre-processing step, defines the parameters of the models which can then, after training and freezing of the model, predict the respective indices during an actual anesthesia procedure when the inputs are presented to the model.

As said, for the processing non-linear models in the shape of fuzzy logic models or quadratic equation models may be employed. However, also other non-linear models may be used.

In the following, by way of example details about ANFIS models and quadratic equation models are provided.

ANFIS model:

A fuzzy logic model may for example be the so-called ANFIS model. In that case, the system uses ANFIS models to combine the parameters, for the definition of the qCON and qNOX indices. The parameters extracted from the EEG signals are used as input to an Adaptive Neuro Fuzzy Inference System (ANFIS).

ANFIS is a hybrid between a fuzzy logic system and a neural network. ANFIS does not assume any mathematical function governing the relationship between input and output. ANFIS applies a data driven approach where the training data decides the behaviour of the system.

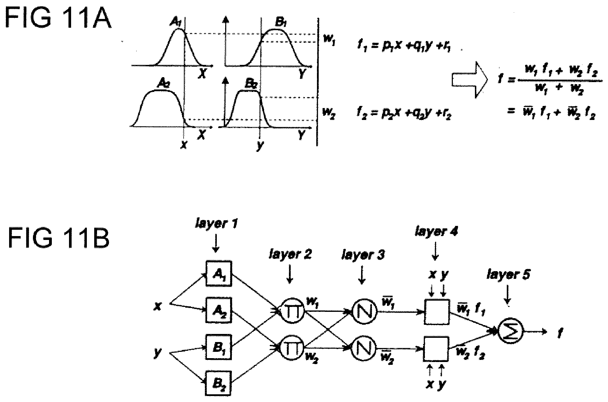

The five layers of ANFIS, shown in Fig. 11A and 11B, have the following functions:

- Each unit in Layer 1 stores three parameters to define a bell-shaped membership function. Each unit is connected to exactly one input unit and computes the membership degree of the input value obtained.
- Each rule is represented by one unit in Layer 2. Each unit is connected to those units in the previous layer, which are from the antecedent of the rule. The inputs into a unit are degrees of membership, which are multiplied to determine the degree of fulfilment for the rule represented.
- In Layer 3, for each rule there is a unit that computes its relative degree of fulfilment by means of a normalisation equation. Each unit is connected to all the rule units in Layer 2.
- The units of Layer 4 are connected to all input units and to exactly one unit in Layer 3. Each unit computes the output of a rule.

- An output unit in Layer 5 computes the final output by summing all the outputs from Layer 4.

Standard learning procedures from neural network theory are applied in ANFIS. Back-propagation is used to learn the antecedent parameters, i.e. the membership functions, and least squares estimation is used to determine the coefficients of the linear combinations in the rules' consequents. A step in the learning procedure has two passes. In the first pass, the forward pass, the input patterns are propagated, and the optimal consequent parameters are estimated by an iterative least mean squares procedure, while the antecedent parameters are fixed for the current cycle through the training set. In the second pass (the backward pass) the patterns are propagated again, and in this pass back-propagation is used to modify the antecedent parameters, while the consequent parameters remain fixed. This procedure is then iterated through the desired number of epochs. If the antecedent parameters initially are chosen appropriately, based on expert knowledge, one epoch is often sufficient as the LMS algorithm determines the optimal consequent parameters in one pass and if the antecedents do not change significantly by use of the gradient descent method, neither will the LMS calculation of the consequents lead to another result. For example in a 2-input, 2-rule system, rule 1 is defined by

$$if\ x\ is\ A\ and\ y\ is\ B\ then\ f_1 = p_1x + q_1y + r_1$$

where *p, q* and *r* are linear, termed consequent parameters or only consequents. Most common is *f* of first order as higher order Sugeno fuzzy models introduce great complexity with little obvious merit.

The inputs to the ANFIS system are fuzzified into a number of predetermined classes. The number of classes should be larger or equal two. The number of classes can be determined by different methods. In traditional fuzzy logic the classes are defined by an expert. The method can only be applied if it is evident to the expert where the landmarks between two classes can be placed. ANFIS optimizes the position of the landmarks, however the gradient descent method will reach its minimum faster if the initial value of the parameters defining the classes is close to the optimal values. By default, ANFIS initial landmarks are chosen by dividing the interval from minimum to maximum of all data into n equidistant intervals, where n is the number of classes. The number of classes could also be chosen by plotting the data in a histogram and visually deciding for an adequate number of classes, by ranking as done by FIR, through various clustering methods or Markov models. The ANFIS default was chosen for this invention and it showed that more than three classes resulted in instabilities during the validation phase, hence either two or three classes were used.

Both the number of classes and number of inputs add to the complexity of the model, i.e., the number of parameters. For example, in a system with four inputs each input may be fuzzified into three classes consisting of 36 antecedent (non-linear) and 405 consequent (linear) parameters, calculated by the following two formulas:

antecedents= number of classes x number of inputs x 3

consequents = number of classes number of inputs x (number of inputs +1)

The number of input-output pairs should in general be much larger (at least a factor 10) than the number of parameters in order to obtain a meaningful solution of the parameters.

A useful tool for ensuring stability is the experience obtained by working with a certain neuro-fuzzy system such as ANFIS in the context of a particular data set, and testing with extreme data for example obtained by simulation

ANFIS uses a Root Mean Square Error (RMSE) to validate the training result and from a set of validation data the RMSE validation error can be calculated after each training epoch. One epoch is defined as one update of both the antecedent and the consequent parameters. An increased number of epochs will in general decrease the training error.

QUADRATIC MODEL

Alternatively, quadratic equation models may be used for the models M1, M2. In that case, the system uses quadratic models to combine the parameters for the definition of the qCON and qNOX indices. Parameters extracted from the EEG signals are used as inputs to a quadratic model.

The output indexes are derived from quadratic generalized models that use as inputs data extracted from the EEG. Such a model contains an independent coefficient called Intercept, one linear term per input, a square term per input and interaction terms between each pair of entries. The model can be expressed as:

$$Output = Intercept + \sum_{i=1}^{n} a_i * Input_i + \sum_{i=1}^{n} b_i * Input_i^2 + \sum_{j=1}^{n}\sum_{i=j+1}^{n} c_{j,i} * Input_i * Input_j$$

Where:

Intercept: intersection or constant term.
Input: input model.
Output: model output.
n: number of model inputs
a: linear terms.
b: square terms
c: interaction terms between inputs.

**List of Reference Numerals**

| | |
|---|---|
| 1 | Rack |
| 2 | Control device |
| 20 | Measurement device |
| 200 | Line |
| 31, 32, 33 | Infusion device |
| 310, 320, 330 | Line |
| 4 | Ventilation device |
| 40 | Mouth piece |
| 400 | Line |
| 41 | Junction |
| 5 | EEG monitor |
| 50 | Electrodes |
| 500 | Line |
| 6 | Display device |
| 7 | Monitor device |
| 70 | Processor device |
| 71 | Memory device |
| 72 | Display device |
| A1-A5 | Compartments |
| C | Correction module |
| D | Drug dosage |
| M1, M2 | Model |
| p | Model |
| P | Patient |
| qCON | Index of consciousness |
| qNOX | Index of nociception |
| qNOXcor | Corrected qNOX index |
| RD | Reference data |
| TD | Training data |

**Claims**

1. A computer-implemented method for training a model (M2) usable to compute an index of nociception (qNOX) related to a nociception effect during a general anesthesia procedure, the method comprising:

    obtaining, during a training phase separate from an actual use of the model (M2) during an anesthesia procedure, clinical data relating to a multiplicity of previous anesthesia procedures,
    deriving training data (TD) from said clinical data,
    deriving reference data (RD) from said clinical data, and
    training said model (M2) using said training data (TD) as input data to the model (M2) and said reference data (RD) as output data to the model (M2), wherein said training includes adjusting the model (M2) according to the training data (TD) and the reference data (RD),

    **characterized in**

    **that** the reference data (RD) is derived from the clinical data using an equation,

$$RD = a \cdot f1(CeRemi) + b \cdot qCON + f3(Resp)$$

including a mathematical term $a \cdot f1(CeRemi)$ whose value is non-linearly variable as a function of a concentration value relating to a drug concentration in a patient during an anesthesia procedure, wherein said function of the concentration value is an exponential function, causing low values of the concentration value to substantially increase the value of the reference data (RD), wherein a is a coefficient, and $f1$ defines said function, and $CeRemi$ is said concentration value,

wherein $b$ is a coefficient, qCON is an index of consciousness, $f3$ is a function and $Resp$ is a patient response parameter and wherein said model mathematical (M2) includes a set of coefficients for computing said index of nociception (qNOX) from input data derived from an encephalography signal (EEG), wherein during said training the coefficients are adjusted to define the model.

2. The method of any of the preceding claims, **characterized in that** said concentration value is time-variable within a particular one of the multiple previous anesthesia procedures.

3. The method of any of the preceding claims, **characterized in that** for deriving the reference data (RD) time-variable reference curves for at least a subset of the multiple previous anesthesia procedures are computed.

4. The method of claim 3,
**characterized in that** that said reference curves are at least one of scaled to a range between 0 and 100 and smoothed by applying a moving average technique.

5. The method of any of the preceding claims, **characterized in that** the model is a fuzzy logic model or a quadratic equation model.

6. A processing system configured to execute a software code implementing the method of any of the preceding claims.

7. A monitor device (7) for computing an index of nociception (qNOX) related to a nociception effect during a general anesthesia procedure, the monitor device (7) comprising:
a processor device (70) configured to compute said index of nociception (qNOX) during an actual anesthesia procedure using a model (M2) and input data which is derived from an encephalography signal (EEG) obtained during said general anesthesia procedure, wherein a value for the index of nociception (qNOX) is obtained as output from the model (M2),
**characterized in**
**that** the processor device (70) is configured to modify said value for the index of nociception (qNOX) obtained from the model (M2) using information related to at least one of an electrooculogram derived from said encephalography signal (EEG), a burst suppression ratio derived from said encephalography signal (EEG) and a near-burst suppression index derived from said encephalography signal (EEG) to obtain a corrected value for the index of nociception (qNOX), wherein the model (M2) is defined, in a training phase prior to the actual anesthesia procedure, by the method of any of the claim 1 to 5.

8. The monitor device (7) of claim 7,
**characterized in that** the processor device (70) is configured to compute said value for the index of nociception (qNOX) in real-time during the actual anesthesia procedure.

9. The monitor device (7) of one of claims 7 and 8,
**characterized in that** the processor device (70) is configured to modify said value for the index of nociception (qNOX) obtained from the model (M2) by in addition applying at least one of a scaling operation and a smoothing operation.

**Patentansprüche**

1. Computerimplementiertes Verfahren zum Trainieren eines Modells (M2), das zur Berechnung eines Nozizeptionsindex (qNOX) verwendet werden kann, der sich auf einen Nozizeptionseffekt während eines allgemeinen Anästhesieverfahrens bezieht, wobei das Verfahren umfasst:

Erhalten von klinischen Daten über eine Vielzahl von früheren Anästhesieverfahren während einer Trainingsphase, die von einer tatsächlichen Verwendung des Modells (M2) während eines Anästhesieverfahrens getrennt ist,

Ableitung von Trainingsdaten (TD) aus den klinischen Daten,

Ableitung von Referenzdaten (RD) aus den klinischen Daten und

Trainieren des Modells (M2) unter Verwendung der Trainingsdaten (TD) als Eingabedaten für das Modell (M2) und der Referenzdaten (RD) als Ausgabedaten für das Modell (M2), wobei das Training das Anpassen des Modells (M2) gemäß den Trainingsdaten (TD) und den Referenzdaten (RD) umfasst,

**dadurch gekennzeichnet,**

**dass** die Referenzdaten (RD) mithilfe einer Gleichung aus den klinischen Daten abgeleitet werden,

$$RD = a\text{-}f1\ (CeRemi) + b\text{-}qCON + f3(Resp)$$

einschließlich eines mathematischen Terms *a-f1 (CeRemi),* dessen Wert als Funktion eines Konzentrationswerts, der sich auf eine Arzneimittelkonzentration in einem Patienten während eines Anästhesieverfahrens bezieht, nichtlinear variabel ist, wobei die Funktion des Konzentrationswerts eine Exponentialfunktion ist, die bewirkt, dass niedrige Werte des Konzentrationswerts den Wert der Referenzdaten (RD) wesentlich erhöhen, wobei a ein Koeffizient ist und *f1* die Funktion definiert, und *CeRemi* der Konzentrationswert ist, wobei *b* ein Koeffizient ist, *qCON* ein Index des Bewusstseins ist, *f3* eine Funktion ist und *Resp* ein Reaktionsparameter des Patienten ist und wobei das mathematische Modell (M2) einen Satz Koeffizienten zur Berechnung des Index der Nozizeption (qNOX) aus Eingangsdaten enthält, die von einem Enzephalographiesignal (EEG) abgeleitet sind, wobei während des Trainings die Koeffizienten angepasst werden, um das Modell zu definieren.

2. Verfahren nach einem der vorhergehenden Ansprüche, **dadurch gekennzeichnet, dass** der Konzentrationswert innerhalb eines bestimmten der mehreren vorangegangenen Anästhesieverfahren zeitvariabel ist.

3. Verfahren nach einem der vorhergehenden Ansprüche, **dadurch gekennzeichnet,** da zur Ableitung der Referenzdaten (RD) zeitvariable Referenzkurven für mindestens eine Teilmenge der mehreren vorangegangenen Anästhesieverfahren berechnet werden.

4. Verfahren nach Anspruch 3,
   **dadurch gekennzeichnet, dass** die Referenzkurven mindestens auf einen Bereich zwischen 0 und 100 skaliert und durch Anwendung einer Technik des gleitenden Durchschnitts geglättet sind.

5. Verfahren nach einem der vorhergehenden Ansprüche, **dadurch gekennzeichnet, dass** das Modell ein Fuzzy-Logik-Modell oder ein quadratisches Gleichungsmodell ist.

6. Verarbeitungssystem, das so konfiguriert ist, dass es einen Softwarecode ausführt, der das Verfahren nach einem der vorhergehenden Ansprüche implementiert.

7. Überwachungsvorrichtung (7) zur Berechnung eines Nozizeptionsindex (qNOX), der sich auf einen Nozizeptionseffekt während eines allgemeinen Anästhesieverfahrens bezieht, wobei die Überwachungsvorrichtung (7) umfasst: eine Prozessorvorrichtung (70), die so konfiguriert ist, dass sie den Nozizeptionsindex (qNOX) während eines tatsächlichen Anästhesieverfahrens unter Verwendung eines Modells (M2) und von Eingabedaten berechnet, die von einem während des allgemeinen Anästhesieverfahrens erhaltenen Enzephalographiesignal (EEG) abgeleitet sind, wobei ein Wert für den Nozizeptionsindex (qNOX) als Ausgabe von dem Modell (M2) erhalten wird,
   **dadurch gekennzeichnet,**
   **dass** die Prozessorvorrichtung (70) so konfiguriert ist, dass sie den Wert für den Nozizeptionsindex (qNOX), der von dem Modell (M2) erhalten wird, unter Verwendung von Informationen modifiziert, die sich auf mindestens eines von einem Elektrookulogramm, das von dem Enzephalographiesignal (EEG) abgeleitet wird, einem von dem Enzephalographiesignal (EEG) abgeleiteten Burst-Suppressionsverhältnis und einem von dem Enzephalographiesignal (EEG) abgeleiteten Near-Burst-Suppressionsindex beziehen, um einen korrigierten Wert für den Nozizeptionsindex (qNOX) zu erhalten, wobei das Modell (M2) in einer Trainingsphase vor dem tatsächlichen Anästhesieverfahren durch das Verfahren nach einem der Ansprüche 1 bis 5 definiert wird.

8. Überwachungsvorrichtung (7) nach Anspruch 7,
   **dadurch gekennzeichnet, dass** die Verarbeitungsvorrichtung (70) so konfiguriert ist, dass sie den Wert für den Nozizeptionsindex (qNOX) in Echtzeit während des tatsächlichen Anästhesieverfahrens berechnet.

9. Überwachungsvorrichtung (7) nach einem der Ansprüche 7 und 8,
**dadurch gekennzeichnet, dass** die Verarbeitungsvorrichtung (70) so konfiguriert ist, dass sie den aus dem Modell (M2) erhaltenen Wert für den Nozizeptionsindex (qNOX) modifiziert, indem sie zusätzlich mindestens eine von einer Skalierungsoperation und einer Glättungsoperation anwendet.

**Revendications**

1. Procédé mis en œuvre informatiquement pour entraîner un modèle (M2) utilisable pour calculer informatiquement un indice de nociception (qNOX) lié à un effet de nociception au cours d'une procédure d'anesthésie générale, le procédé comprenant :

   l'obtention, au cours d'une phase d'entraînement distincte de l'utilisation proprement dite du modèle (M2) au cours d'une procédure d'anesthésie, de données cliniques relatives à une multiplicité de procédures d'anesthésie antérieures,
   le fait de tirer des données d'entraînement (TD) desdites données cliniques,
   le fait de tirer des données de référence (RD) desdites données cliniques et
   l'entraînement dudit modèle (M2) à l'aide desdites données d'entraînement (TD) en tant que données d'entrée du modèle (M2) et desdites données de référence (RD) en tant que données de sortie du modèle (M2), ledit entraînement incluant l'ajustement du modèle (M2) selon les données d'entraînement (TD) et les données de référence (RD),

   **caractérisé en ce que**

   les données de référence (RD) sont tirées des données cliniques à l'aide d'une équation,

$$RD = a \cdot f1(CeRemi) + b \cdot qCON + f3(Resp)$$

   incluant un terme mathématique $a \cdot f1(CeRemi)$ dont la valeur est variable de façon non linéaire en fonction d'une valeur de concentration relative à une concentration de médicament chez un patient au cours d'une procédure d'anesthésie, ladite fonction de la valeur de concentration étant une fonction exponentielle, amenant les faibles valeurs de la valeur de concentration à augmenter sensiblement la valeur des données de référence (RD), $a$ étant un coefficient et $f1$ définissant ladite fonction et $CeRemi$ étant ladite valeur de concentration, $b$ étant un coefficient, $qCON$ étant un indice de conscience, $f3$ étant une fonction et $Resp$ étant un paramètre de réponse du patient et ledit modèle mathématique (M2) incluant un ensemble de coefficients pour calculer informatiquement ledit indice de nociception (qNOX) à partir de données d'entrée tirées d'un signal d'encéphalographie (EEG), les coefficients étant, au cours dudit entraînement, ajustés pour définir le modèle.

2. Procédé selon l'une quelconque des revendications précédentes, **caractérisé en ce que** ladite valeur de concentration est variable dans le temps sur une procédure d'anesthésie particulière parmi les multiples procédures d'anesthésie antérieures.

3. Procédé selon l'une quelconque des revendications précédentes, **caractérisé en ce que** pour tirer les données de référence (RD), des courbes de référence variables dans le temps sont calculées informatiquement pour au moins un sous-ensemble des multiples procédures d'anesthésie antérieures.

4. Procédé selon la revendication 3,
**caractérisé en ce que** lesdites courbes de référence sont mises à l'échelle dans une plage comprise entre 0 et 100 et/ou lissées par application d'une technique de moyenne glissante.

5. Procédé selon l'une quelconque des revendications précédentes, **caractérisé en ce que** le modèle est un modèle à logique floue ou un modèle à équation quadratique.

6. Système de traitement configuré pour exécuter un code logiciel mettant en œuvre le procédé selon l'une quelconque des revendications précédentes.

7. Dispositif de surveillance (7) pour calculer informatiquement un indice de nociception (qNOX) lié à un effet de nociception au cours d'une procédure d'anesthésie générale, le dispositif de surveillance (7) comprenant :
un dispositif processeur (70) configuré pour calculer informatiquement ledit indice de nociception (qNOX) au cours d'une procédure d'anesthésie proprement dite à l'aide d'un modèle (M2) et de données d'entrée qui sont tirées d'un signal d'encéphalographie (EEG) obtenu au cours de ladite procédure d'anesthésie générale, une valeur de l'indice de nociception (qNOX) étant obtenue en tant que sortie du modèle (M2),
**caractérisé en ce que**
le dispositif processeur (70) est configuré pour modifier ladite valeur de l'indice de nociception (qNOX) obtenue à partir du modèle (M2) à l'aide d'informations liées à au moins l'un d'un électro-oculogramme tiré dudit signal d'encéphalographie (EEG), d'un ratio de suppression de salves tiré dudit signal d'encéphalographie (EEG) et d'un indice de quasi-suppression de salves tiré dudit signal d'encéphalographie (EEG) pour obtenir une valeur corrigée de l'indice de nociception (qNOX), le modèle (M2) étant défini, dans une phase d'entraînement avant la procédure d'anesthésie proprement dite, par le procédé selon l'une quelconque des revendications 1 à 5.

8. Dispositif de surveillance (7) selon la revendication 7,
**caractérisé en ce que** le dispositif processeur (70) est configuré pour calculer informatiquement ladite valeur de l'indice de nociception (qNOX) en temps réel au cours de la procédure d'anesthésie proprement dite.

9. Dispositif de surveillance (7) selon l'une des revendications 7 et 8,
**caractérisé en ce que** le dispositif processeur (70) est configuré pour modifier ladite valeur de l'indice de nociception (qNOX) obtenue à partir du modèle (M2) en appliquant de plus au moins l'une d'une opération de mise à l'échelle et d'une opération de lissage.

FIG 1

FIG 2

FIG 3

# FIG 4

EEG → M1 → qCON

70

# FIG 5

EEG → M2 → qNOX → C → qNOXcor

70

# FIG 6

# FIG 7

# FIG 8

# FIG 9

# FIG 10

FIG 11A

$$f_1 = p_1 x + q_1 y + r_1$$

$$f_2 = p_2 x + q_2 y + r_2$$

$$f = \frac{w_1 f_1 + w_2 f_2}{w_1 + w_2}$$

$$= \overline{w}_1 f_1 + \overline{w}_2 f_2$$

FIG 11B

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017012622 A1

- WO 2014173558 A1

**Non-patent literature cited in the description**

- **E. W. JENSEN et al.** Monitoring hypnotic effect and nociception with two EEG-derived indices, qNOX and qCON, during general anesthesia. *Acta Anaesthesiologica Scandinavica*, 2014, vol. 58, 933-941